# EUROPEAN PATENT APPLICATION

(11) **EP 2 308 385 A1**
(43) Date of publication of application: **13.04.2011**
(21) Application number: 10251775.2
(22) Date of filing: 08.10.2010
(51) Int. Cl.: A61B 17/04, A61B 17/00, A61B 17/12

(54) **Atraumatic tissue anchor**

(30) Priority: 09.10.2009 US 250066 P; 04.10.2010 US 896939
(71) Applicant: Tyco Healthcare Group LP, New Haven, CT 06511 (US)
(72) Inventor: Fischvogt, Gregory, Hamden, CT 06514 (US)
(74) Representative: Soames, Candida Jane

(57) **Abstract**

A tissue anchor assembly includes a quantity of material (20), an anchoring object (30) and a securing member (28). The quantity of material is configured for positioning adjacent a face of tissue (102). The anchoring object is configured for positioning adjacent an opposite face of tissue (104). The securing member is configured to fixedly retain the quantity of material about the anchoring object to thereby secure tissue between the quantity of material and the anchoring object.

## Description

### CROSS-REFERENCE TO RELATED APPLICATION

The present application claims the benefit of and priority to U.S. Provisional Application Serial No. 61/250,066 filed on October 9, 2009, the entire contents of which are incorporated by reference herein.

### BACKGROUND

### Technical Field

The present disclosure relates to a tissue anchor and, more particularly, to an atraumatic tissue anchor assembly and method for attaching a tissue anchor to tissue.

### Description of Related Art

As a result of recent technological improvements in surgical instruments, surgical procedures that were previously performed in a conventional or open fashion are now routinely performed using minimally-invasive surgical techniques (e.g., endoscopic, laparoscopic, etc.). Minimally-invasive surgical procedures are less invasive as compared to conventional surgical procedures and, thus, minimize trauma to the patient and reduce patient recovery time.

In endoscopic and laparoscopic surgical procedures, it is often necessary to provide instrumentation to move or manipulate tissue and organs located in or near the area of operation. Generally, laparoscopic surgical procedures involve the introduction of a gas, e.g., carbon dioxide, to insufflate a body cavity, e.g., the abdomen, to provide a working area for the surgeon. More particularly, a trocar device is utilized to puncture the peritoneum to provide an access port by way of a cannula through the abdominal wall. A tube supplying an insufflation gas may then be inserted through the cannula to insufflate the abdomen. The cannula typically includes a fluid-tight seal for maintaining the body cavity in the insufflated condition. Generally, a trocar/cannula is placed through the abdominal wall for introduction of each piece of surgical instrumentation which is necessary to carry out the surgical procedure. In this manner, the surgeon may view the surgical site through an endoscope provided through a first trocar/cannula, and may utilize a second trocar/cannula to introduce a surgical instrument such as a grasper, scissor, clip applier, stapler or any other surgical instrument which may be necessary during the particular surgical procedure.

Although the insufflation gas expands the abdomen to permit the surgeon to view the surgical site, it is often necessary to manipulate internal organs or tissues within the surgical site to provide a clear path to the surgical objective. In the past, surgeons have fastened anchors internally to soft tissue, or viscera, during surgical procedures using hooks or other sharp objects. As can be appreciated, the use of such hooks and other sharp objects may cause great trauma to a patient during the insertion, relocation and/or removal of these anchors.

### SUMMARY

In accordance with the present disclosure, a tissue anchor assembly is provided. The tissue anchor assembly includes a quantity of material, e.g., a suture, configured for positioning adjacent a dace of tissue. The tissue anchor assembly further includes an anchoring object configured for positioning adjacent an opposite face of tissue. A securing member is configured to fixedly retain the quantity of material about the anchoring object, thereby securing tissue between the quantity of material and the anchoring object.

In one embodiment, the quantity of material is a suture having first and second ends. The first end of the suture is knotingly engaged to the suture to form a suture loop. The first end of the suture may be knotingly engaged to the suture to form a cinch knot capable of translating along the suture to cinch the suture loop around tissue disposed therethrough. The second end of the suture may be moved, or manipulated to retract tissue.

In another embodiment, the anchoring object is generally spherical in shape. Further, the anchoring object and/or the quantity of material may be formed from a bio-absorbable material such that at the anchoring object and/or the quantity of material may be left behind after completion of the surgical procedure.

In accordance with the present disclosure, another embodiment of a tissue anchor assembly is provided. The tissue anchor assembly includes a cinch loop and an anchoring object. The cinch loop is configured for positioning adjacent a face of tissue and is transitionable between a first position and a second position. In the first position, the cinch loop defines a first diameter. In the second position, the cinch loop defines a second diameter different from the first diameter. The anchoring object has a diameter smaller than the first diameter of the cinch loop, e.g., the diameter of the anchoring object is greater than that of the cinch loop when the cinch loop is in the first position, but greater than the second diameter of the cinch loop, e.g., the diameter of the anchoring object is greater than that of the cinch loop when the cinch loop is in the second position. The anchoring object configured for positioning adjacent an opposite face of tissue. When the cinch loop is in the first position, the anchoring object is advanceable partially (or entirely) through the cinch loop to urge tissue through the cinch loop. As such, when the anchoring object is advanced through the cinch loop, tissue is similarly advanced therethrough such that tissue is disposed between the cinch loop and the anchoring object. When the cinch loop is moved to the second position, with the anchoring object and tissue disposed therethrough, the anchoring object and tissue are inhibited from passing back through the cinch loop. Accordingly, tissue is fixedly engaged between the anchoring object and the cinch loop when the cinch loop is moved to the second position.

A tissue anchor assembly in accordance with yet another embodiment of the present disclosure includes a ring structure and an expandable member. The ring structure is configured for positioning adjacent a face of tissue. The expandable member is configured for positioning adjacent an opposite face of tissue. The expandable member is expandable from a first position to a second position for fixedly retaining tissue disposed through the ring structure.

In one embodiment, a retraction suture is attached to the ring structure to permit retraction of tissue.

In another embodiment, the expandable member includes a cam fastener having an expandable tip portion. The tip portion of the cam fastener is expandable from a first position for passing through the ring structure to a second position whereby the tip portion is expanded radially outward to secure tissue disposed through the ring structure.

In yet another embodiment, the expandable member, e.g., a balloon, is expandable by introducing an inflation fluid into a chamber defined within the expandable member.

In accordance with another embodiment of the present disclosure, a tissue anchor assembly is provided. The tissue anchor assembly includes a ring and an expandable member. The ring is configured for positioning adjacent a face of tissue and defines an opening therethrough. The expandable member is configured for positioning adjacent an opposite face of tissue and is expandable from a first position to a second position. In the first position, the expandable member is partially (or entirely) advanceable through the opening of the ring to urge tissue through the opening of the ring. As such, when the expandable member is advanced through the opening of the ring, tissue is similarly advanced to be disposed between the ring and the expandable member. In the second position, the expandable member is inhibited from passing through the opening of the ring. Accordingly, when the expandable member is transitioned to the second position when disposed through the ring, tissue is fixedly engaged between the expandable member and the ring.

A method of attaching a tissue anchor to tissue is also provided in accordance with the present disclosure. The method includes providing first and second components, e.g., a cinch loop and an anchoring object or a ring and an expandable member. The first component has an opening defined therethrough. The second component is partially (or entirely) insertable through the opening of the first component. One or both of the first and second components is transitionable between a first position and a second position. The first component is positioned adjacent a face of tissue, while the second component is positioned adjacent an opposite face of tissue. The second component is then advanced through the opening of the first component to urge tissue through the opening such that tissue is disposed between the first and second components. Next, the first and/or the second components are transitioned to the second position to fixedly retain tissue between the first and second components.

In one embodiment, the first and/or second components may then be manipulated to retract tissue fixedly retained therebetween. In order to remove the tissue anchor, the first and/or the second components may be transitioned back to the first position to permit the second component and tissue to be released, or disengaged from the first component.

The invention may be described by reference to the following numbered paragraphs:-

A tissue anchor assembly according to the present invention for use in a method of attaching a tissue anchor to tissue, comprising the steps of: providing first and second components, the first component having an opening defined therethrough, the second component being at least partially insertable through the opening of the first component, at least one of the first and second components being transitionable between a first position and a second position; positioning the first component adjacent a face of tissue; positioning the second component adjacent an opposite face of tissue; advancing the second component through the opening of the first component to urge tissue through the opening of the first component such that tissue is disposed between the first and second components; and transitioning the at least one of the first and second components to the second position to fixedly retain tissue between the first and second components.

A tissue anchor assembly according to paragraph [0018], further comprising the step of manipulating at least one of the first and second components for retracting tissue fixedly retained therebetween;

A tissue anchor assembly according to paragraph [0018] or [0019] further comprising the step of transitioning the at least one of the first and second components back to the first position to permit the second component and tissue to be released from the first component.

### BRIEF DESCRIPTION OF THE DRAWINGS

Various embodiments of the subject tissue anchor assembly are described herein with reference to the drawings wherein:

Fig. 1 is a perspective view of a tissue anchor assembly in accordance with one embodiment of the present disclosure shown anchored to tissue;

Fig. 2 is a side, cross-sectional view of the tissue anchor assembly of Fig. 1 shown anchored to tissue;

Fig. 3 is a top view of a tissue anchor assembly in accordance with another embodiment of the present disclosure shown in position to be anchored to tissue;

Fig. 4 is a side, cross-sectional view of the tissue anchor assembly of Fig. 3 shown in position to be anchored to tissue;

Fig. 5 is a top view of the tissue anchor assembly of Fig. 3, shown anchored to tissue;

Fig. 6 is a side, cross-sectional view of the tissue anchor assembly of Fig. 3 shown anchored to tissue;

Fig. 7 is a top view of a tissue anchor assembly in accordance with yet another embodiment of the present disclosure shown in position to be anchored to tissue;

Fig. 8 is a side, cross-sectional view of the tissue anchor assembly of Fig. 7 shown in position to be anchored to tissue;

Fig. 9 is a top view of the tissue anchor assembly of Fig. 7 shown anchored to tissue; and

Fig. 10 is a side, cross-sectional view of the tissue anchor assembly of Fig. 7 shown anchored to tissue.

### DETAILED DESCRIPTION

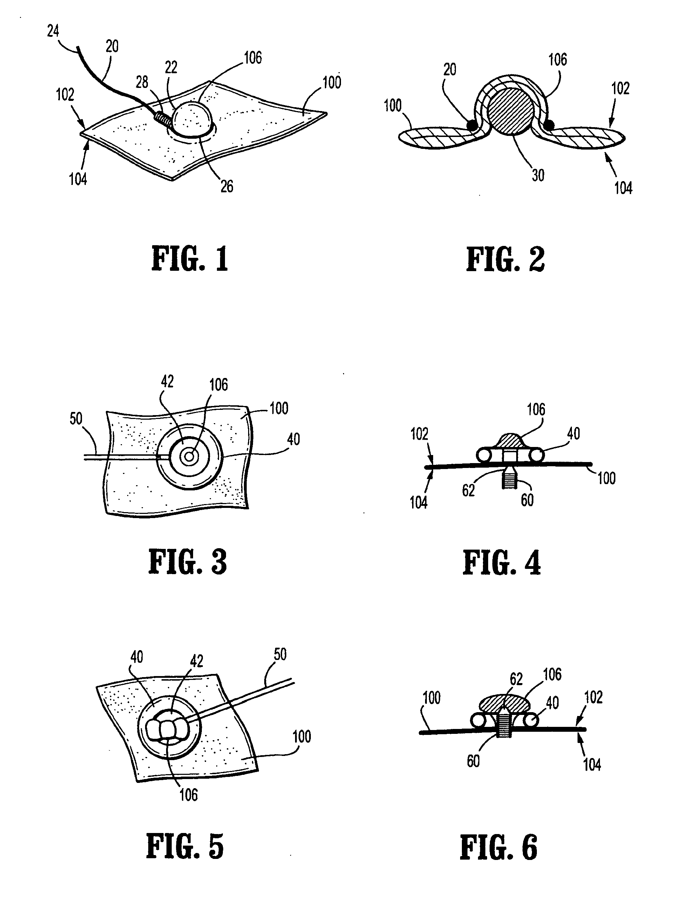

Turning now to Fig. 1, one embodiment of a tissue anchor assembly is shown wherein suture 20 is positioned adjacent a first face 102 of tissue 100 and wherein an anchoring object 30 (see Fig. 2) is disposed adjacent a second face 104 of tissue 100. Suture 20 includes a first end 22 that is knotingly engaged with suture 20 via cinch knot 28 to form suture loop 26. Cinch knot 28 is slidable along suture 20 to selectively tighten suture loop 26 about a portion of tissue 106 disposed therethrough. Second, or free end 24 of suture 20 is manually manipulatable to retract tissue 106 cinched through suture loop 26.

As shown in Fig. 2, anchoring object 30 defines a generally smooth, spherical configuration, although other configurations are contemplated. Smooth, rounded or spherical configurations help reduce stress concentrations within tissue 100 and help distribute the load more evenly throughout a larger area of tissue 100, thereby reducing the likelihood of damaging tissue 100. Anchoring object 30 and/or suture 20 may be made from any suitable bio-absorbable material, such that anchoring object 30 and/or suture 20 may be left behind after the surgical procedure is complete. Suitable bio-absorbable materials include, but are not limited to polyglycolides, polylactides and copolymers thereof as well as natural materials (e.g., gut).

In operation, suture 20 is positioned adjacent first face 102 of tissue 100 and anchoring object 30 is placed adjacent opposite face 104 of tissue 100 such that anchoring object 30 and suture loop 26 are disposed adjacent on another with tissue 100 therebetween. Once the suture 20 and anchoring object 30 are positioned as described above, anchoring object 30 is moved toward tissue 100 and suture loop 26 such that a portion 106 of tissue 100 is urged through suture loop 26 and such that at least a portion of anchoring object 30 is also urged through suture loop 26, as shown in Fig. 1. Alternatively, where tension on tissue 100 is greater, placement of the anchoring object 30 adjacent opposite face 104 of tissue 100 may create a bulge in tissue 100 in the vicinity of anchoring object 30, thereby obviating the need to move anchoring object 30 toward tissue 100. In this situation, the bulge created by the tension in tissue 100 and the presence of anchoring object 30 would force a portion 106 of tissue 100 through suture loop 26.

As shown in Fig. 2, anchoring object 30 is advanced toward tissue 100 to urge portion 106 of tissue 100 through suture loop 26. Anchoring object 30 is advanced further through suture loop 26 such that portion 106 of tissue 100 and anchoring object 30 are substantially disposed through suture loop 26, i.e., such that suture loop 26 substantially surrounds both anchoring object 30 and portion 106 of tissue 100. Once anchoring object 30 has been position as described above and as shown in Fig. 2, suture loop 26 may be cinched around tissue 106 by tightening cinch knot 28. Cinch knot 28 is tightened until suture loop 26 has been sufficiently reduced to inhibit object 30 from retreating back away from tissue 100, i.e., cinch knot 28 is tightened such that anchoring object 30 is inhibited from passing through suture loop 26. In this cinched, or tightened position, suture loop 26 retains anchoring object 30 and portion 106 of tissue 100 surrounding anchoring object 30, in a fixed position. As mentioned above, suture loop 26 is sufficiently small to inhibit anchoring object 30 from retreating, while tissue 106 inhibits anchoring object 30 from advancing further. Similarly, suture loop 26 is fixedly surrounded by anchoring object 30 on one side and by face 102 of tissue 100 on the other side, thereby securing suture loop 26 in place.

Once suture 20 is fixedly secured about tissue 100 and anchoring object 30, free end 24 of suture 20 may be manipulated, or pulled to retract tissue 100. Accordingly, tissue 100 may be retracted from its at-rest position, in the direction of manipulation of free end 24 of suture 20, allowing a surgeon to operate on underlying or nearby tissue. As can be appreciated, with suture loop 26 tightened sufficiently about portion 106 of tissue 100, suture 20 may be manipulated to retract tissue 100, rather than slide, or translate with respect to tissue 100 due to the positioning of anchoring object 30 on one side thereof and the positioning of tissue 100 on the other side thereof. This configuration also helps minimize slippage of suture 20 during retraction.

With reference now to Figs. 3-6, another embodiment of a tissue anchor assembly is shown generally including a ring 40 defining bore 42 therethrough, a retraction suture 50, and a cam fastener 60. Ring 40 is positioned adjacent a first face 102 of tissue 100. Ring 40 may be formed from any suitable bio-compatible material and may be a rigid or semi-rigid ring 40. A retracting suture 50 is looped around, or otherwise attached to ring 40 for retraction of tissue 100, as will be described in greater detail below. Alternatively, an aperture (not shown) defined through ring 40 may be provided to permit passage of retraction suture 50 therethrough for securing retraction suture 50 to ring 40.

As shown in Fig. 4, cam fastener 60 includes a tip portion 62 that is positioned adjacent an opposite face 104 of tissue 100. Tip portion 62 of cam fastener 60 is transitionable between a contracted state defining a first diameter allowing cam fastener 60 to pass through bore 42 of ring 40, and an expanded state defining a second diameter greater than a diameter of bore 42 of ring 40 such that cam fastener 60 is inhibited form passing through bore 42 of ring 40. In use, cam fastener 60 is initially disposed in the contracted state and is positioned adjacent bore 42 of ring 40 with tissue 100 disposed therebetween. More particularly, tip portion 62 of cam fastener 60 is positioned adjacent portion 106 of tissue 100 on one side, or face 104 thereof, while ring 40 is positioned adjacent portion 106 of tissue 100 on the opposite side, or face 102 thereof. Tip portion 62 of cam fastener 60 may define a blunt tip to minimize tissue trauma during contact with tissue 100. Further, cam fastener 60 may be disposed on the distal portion of a surgical instrument (e.g., a blunt tipped obturator) and/or may be separable from the surgical instrument (not shown).

In operation, with cam fastener 60 in the contracted state, cam fastener 60 is advanced toward tissue 100 and ring 40, as shown in Fig. 4, to urge portion 106 of tissue 100 through bore 42 of ring 40. As cam fastener 60 is advanced further, as shown in Figs. 5 and 6, tissue 100 is further urged through bore 42 of ring 40 and, eventually, tip portion 62 of cam fastener 60 is advanced through bore 42 of ring 40. Once tip portion 62 of cam fastener is disposed through bore 42 of ring 40, tip portion 62 may be expanded radially outward, to the expanded state, thereby urging portion 106 of tissue 100, which is disposed through ring 40, to expand radially outward. Tip portion 62 of cam fastener may be configured to expand from the contracted state to the expanded state upon insertion of a pin (not shown) into cam fastener 60 or may be expandable by any other suitable activation mechanism (not shown). As mentioned above, when tip portion 62 of cam fastener 60 is transitioned to the expanded state, tip portion 62 is expanded radially outward to define a diameter that is greater than the diameter of bore 42 of ring 40. As such, with tip portion 62 of cam fastener 60 in the expanded position (see Fig. 6), cam fastener 60 and, thus portion 106 of tissue 100 are inhibited from retreating back through bore 42 of ring 40. Thus, as can be appreciated, the expansion of cam fastener 60 to the expanded state fixedly secures ring 40 between tissue 100 and the expanded tip portion 62 of cam fastener 60 (and portion 106 of tissue 100 disposed therearound). With ring 40 secured in position, as described above, retraction suture 50 may manipulated to manipulate ring 40, thereby retracting tissue 100 and allowing the surgeon to access tissue underlying and/or surrounding tissue 100.

When it is desired to remove the tissue anchor assembly, the pin (not shown) may be removed from cam fastener 60, allowing tip portion 62 of cam fastener 60 to return to the contracted state. In instances where other activation structures are employed to expand tip portion 62 of cam fastener 60, it is envisioned that these structures also be reversible to allow tip portion 62 to return to the contracted state for removal. Once the diameter of tip portion 62 is reduced back to the first diameter, i.e., once the diameter of tip portion 62 of cam fastener is once again smaller than the diameter of bore 42 of ring 40, tip portion 62 and, thus, portion 106 of tissue 100 may be passed back through bore 42 of ring 40, disengaging the tissue anchor assembly, e.g., ring 40 and cam fastener 60, from tissue 100. Cam fastener 60 and ring 40 may then be removed from the surgical site, allowing tissue 100 to return to its at-rest position.

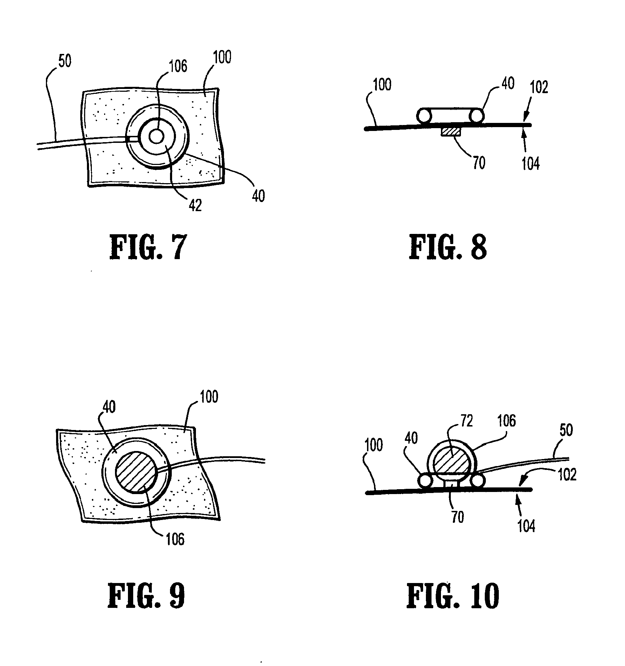

In another embodiment of a tissue anchor assembly, as shown in Figs. 7-10, an inflatable balloon 70 may be provided in place of cam fastener 60. Similar to cam fastener 60, inflatable balloon 70 is transitionable between a contracted state and an expanded state and is positionable adjacent bore 42 of ring 40. More particularly, in use, inflatable balloon 70 is positioned adjacent face 104 of tissue 100, while ring 40 is positioned adjacent face 102 of tissue 100 such that balloon 70 and ring 40 are disposed adjacent one another. Upon alignment of balloon 70 and ring 40 about tissue 100, an internal chamber (not shown) defined within balloon 70 may be supplied with an inflation fluid, e.g., CO₂, to expand balloon 70 from the contracted state to the expanded state, urging balloon 70 through bore 42 of ring 40. As balloon 70 is urged through bore 42 of ring 40, portion 106 of tissue 100 is similarly urged through bore 42 of ring 40. As shown in Fig. 10, balloon 70 may be inflated such that expanded portion 72 of balloon 70 and, thus, portion 106 of tissue 100 are substantially disposed through bore 42 of ring 40. Upon further inflation of balloon 70 toward the expanded state, the diameter of balloon 70 (and portion 106 of tissue 100) eventually exceeds the diameter of bore 42 of ring 40 such that balloon 70 and portion 106 of tissue 100 are inhibited from passing back through bore 42 of ring 40. Accordingly, with balloon 70 in the expanded state, balloon 70 and portion 106 of tissue 100 are fixedly retained in position by ring 40.

Balloon 70 may include a check valve (not shown) to allow balloon 70 to be disconnected from the inflation source upon reaching the expanded state. The check valve (not shown) operates to retain the inflation fluid within the chamber defined within balloon 70 and, thus, retains balloon 70 in the expanded state. Once balloon 70 and portion 106 of tissue 100 are fixedly secured in position by ring 40, retraction suture 50 may be used to retract tissue 100, as described above.

When it is desired to remove the tissue anchor assembly, balloon 70 may be deflated via opening a release valve (not shown) or may be deflated by any other suitable means. As balloon 70 is deflated, the diameter of expanded portion 72 of balloon 70 decreases, eventually reaching a diameter smaller than that of bore 42 of ring 40, thereby allowing balloon 70 and, thus, portion 106 of tissue 100, to retreat back through bore 42 of ring 40, disengaging the tissue anchor assembly, e.g., balloon 70 and ring 40, from tissue 100.

From the foregoing and with reference to the various figure drawings, those skilled in the art will appreciate that certain modifications can also be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. A tissue anchor assembly, comprising:
a quantity of material configured for positioning adjacent a face of tissue;
an anchoring object configured for positioning adjacent an opposite face of tissue; and
a securing member configured to fixedly retain the quantity of material about the anchoring object, thereby securing tissue between the quantity of material and the anchoring object.

2. The tissue anchor assembly according to claim 1, wherein the quantity of material is a suture having a first end and a second end, the first end of the suture knotingly engaged with the suture to form a suture loop.

3. The tissue anchor assembly according to claim 2, wherein the first end of the suture comprises a cinch knot, the cinch knot engaging the first end of the suture to the suture such that the cinch knot is moveable along the suture to define a suture loop to cinch around tissue disposed through the suture loop.

4. The tissue anchor assembly according to claim 2 or 3, wherein the second end of the suture is moveable to retract tissue.

5. The tissue anchor assembly according to any preceding claim, wherein the anchoring object is generally spherical.

6. The tissue anchor assembly according to any preceding claim, wherein at least one of the anchoring object and the quantity of material are formed from a bio-absorbable material such at least one of the anchoring object and the quantity of material can be left behind after a surgical procedure.

7. A tissue anchor assembly, comprising:
a cinch loop configured for positioning adjacent a face of tissue, the cinch loop being transitionable between a first position, wherein the cinch loop defines a first diameter, and a second position, wherein the cinch loop defines a second diameter different from the first diameter;
an anchoring object having a diameter smaller than the first diameter of the cinch loop and greater than the second diameter of the cinch loop, the anchoring object configured for positioning adjacent an opposite face of tissue; and
wherein, when the cinch loop is in the first position, the anchoring object is advanceable at least partially through the cinch loop to urge tissue through the cinch loop such that tissue is disposed between the cinch loop and the anchoring object, and wherein, when the cinch loop is moved to the second position with the anchoring object and tissue disposed therethrough, the anchoring object and tissue are inhibited from passing back through the cinch loop, such that tissue is fixedly engaged between the anchoring object and the cinch loop.

8. A tissue anchor assembly, the tissue anchor assembly comprising:
a ring structure configured for positioning adjacent a face of tissue; and
an expandable member configured for positioning adjacent an opposite face of tissue, the expandable member being expandable from a first, contracted position to a second, expanded position for fixedly retaining tissue disposed through the ring structure.

9. The tissue anchor assembly according to claim 8, further comprising a retraction suture attached to the ring structure and adapted for retracting tissue.

10. The tissue anchor assembly according to claim 8 or claim 9, wherein the expandable member includes a cam fastener having an expandable tip portion, the tip portion of the cam fastener being expandable from a first position for passing through the ring structure to a second position whereby the tip portion is expanded radially outward to secure tissue disposed through the ring structure.

11. The tissue anchor assembly according to claim 8 or 9, wherein the expandable member is expandable by introducing an inflation fluid into a chamber defined within the expandable member.

12. A tissue anchor assembly, comprising:
a ring configured for positioning adjacent a face of tissue, the ring defining an opening therethrough; and
an expandable member configured for positioning adjacent an opposite face of tissue, the expandable member being expandable from a first position, wherein the expandable member is at least partially advanceable through the opening of the ring to urge tissue through the opening of the ring such that tissue is disposed between the ring and the expandable member, to a second position, wherein the expandable member is inhibited from passing through the opening of the ring, such that tissue is fixedly engaged between the expandable member and the ring.

13. A tissue anchor assembly according to any preceding claim for use in a method of attaching a tissue anchor to tissue, comprising the steps of:
providing first and second components, the first component having an opening defined therethrough, the second component being at least partially insertable through the opening of the first component, at least one of the first and second components being transitionable between a first position and a second position;
positioning the first component adjacent a face of tissue;
positioning the second component adjacent an opposite face of tissue;
advancing the second component through the opening of the first component to urge tissue through the opening of the first component such that tissue is disposed between the first and second components; and
transitioning the at least one of the first and second components to the second position to fixedly retain tissue between the first and second components.

14. A tissue anchor assembly according to claim 13, further comprising the step of manipulating at least one of the first and second components for retracting tissue fixedly retained therebetween.

15. A tissue anchor assembly according to claim 13 or claim 14 further comprising the step of transitioning the at least one of the first and second components back to the first position to permit the second component and tissue to be released from the first component.
